# EUROPEAN PATENT SPECIFICATION

(11) **EP 2 766 383 B1**
(45) Date of publication and mention of the grant of the patent: **08.08.2018**
(21) Application number: 12791864.7
(22) Date of filing: 12.10.2012
(51) Int. Cl.: C07K 4/12, A23J 1/12, C12P 21/06

(54) **PEPTIDES FROM FISH GELATINE**
PEPTIDE AUS FISCHGELATINE
PEPTIDES DE GÉLATINE DE POISSON

(30) Priority: 14.10.2011 IN 2960DE2011
(43) Date of publication of application: 20.08.2014
(73) Proprietor: Council of Scientific and Industrial Research, New Delhi 110 001 (IN)
(72) Inventor: GADRE, Ramchandra, Vithal, Pune 411008 (IN); JOGDAND, Vithal, Venkatrao, Pune 411008 (IN); NENE, Sanjay, Narayan, Pune 411008 (IN)
(74) Representative: Cabinet Plasseraud
(86) International application number: PCT/IN2012/000678
(87) International publication number: WO 2013/054363

(56) References cited:
- EP-A1- 1 413 614
- KR-A- 20090 106 807
- US-A1- 2006 269 987

## Description

### TECHNICAL FIELD OF THE INVENTION

The invention relates to a process of preparing low molecular weight peptides from gelatine, as defined in the claims. More particularly, the present invention relates to an enzymatic breakdown of gelatine to peptides and obtaining such peptides with low ash content.

### BACKGROUND AND PRIOR ART OF THE INVENTION

Collagen is one of the major constituents of connective tissues of animal, bird, fish, and so on. Gelatine, a denatured form of collagen, has been prepared in industrial scale from these materials. The gelatine based products have a long history as food ingredients. Gelatine is the product of denaturation or disintegration of collagen. Gelatine, because of its high molecular weight and helical structure, is not easily digested and products called "peptides" derived through hydrolysis of gelatine are becoming popular as nutritional supplements.

In aqueous solutions, gelatine is a mixture of different polypeptide chains including α-chains, β (dimers of α-chain) and γ (trimers of α-chain) components. Gelatine is being widely used in food, drug and cosmetic industries as stabilizing, thickening and gelling agent. The most important properties that makes gelatine very favourable to be used in the food industry is its low melting temperature, in which it is molten at human body temperature and higher gel strength when compared to other common gelling agents. Gelatine currently extracted from bovine skin and bones became unpopular in the world because of the fear of diseases found in the animal world.

Fish skin and bones are waste generated in fish processing industry. Normally the waste of fish processing industry will be sold at very low price. The fish skin contains collagen which is a structural protein. Gelatine is made from collagen and is a large molecular weight protein which when hydrolyzed, generates peptides of small molecular weight (less than 2500 Da) that are easily absorbed by human body. These collagen peptides are also used in cosmetic industry as skin tonners. Gelatine is made currently from bovine skin but because of bovine spongiform encephalopathy (mad cow disease) alternative sources of gelatine are being preferred. Gelatine extraction from fish skin and bones and its conversion to peptides will effectively utilize this bio-resource and may add value to the fish processing industry.

Therefore, gelatine obtained from fish can become a substitute for bovine gelatine because it is free of the bovine disease causing agents. Although fish can be used as a source of gelatine there are issues of odour, ash content and undesired molecular weight of peptides. There is ample literature available in the art to extract the small peptide molecules from gelatine by digesting the same with an enzyme.

References may be made to an article titled "Mass spectrometric detection of marker peptides in tryptic digests of gelatin" reported by Guifeng Zhang in Food Hydrocolloids 23 (2009) 2001-2007, discloses the tryptic digestion of bovine gelatin in presence of NH₄HCO₃ (pH 8.0). However, trypsin used as the enzyme for hydrolysis is not an aggressive protease, resulting in poor yields of the peptides. Trypsin an enzyme of animal origin has an optimal activity at 37°C. Use of 37°C in hydrolysis processes can lead to microbial proliferation and cause spoilage of the product. The above mentioned reference also required 10 hours for hydrolysis that is too long.

Document KR20090106807A discloses a process for preparing low MW peptides derived from fish gelatin employing the same type of enzyme (B. licheniformis derived protease). The document is silent on desalting the clarified solution through an ultrafiltration membrane in a diafiltration mode. Furthermore, the enzymatic digestion is carried out for 24 hours.

### OBJECTS OF THE INVENTION

Main object of the present invention is to obtain low molecular weight peptides from gelatine in good yields through an enzymatic hydrolysis process with low ash content. Another object of the present invention is to provide a process that result in the complete hydrolysis of gelatine.

### SUMMARY OF THE INVENTION

Accordingly, present invention provides A process for preparing peptide powder of low molecular weight comprising the steps of:
a. clarifying 0.5 to 6.5% w/v fish gelatine solution by adjusting pH upto 7 followed by centrifuging the solution followed by membrane filtration to make the solution particle-free by discarding the solid particles;
b. desalting the solution as clarified in step (a) through ultrafiltration membrane at temperature in the range of 20 to 55°C in a diafiltration mode till the salt content decreases below 0.05% (w/v) followed by discarding the permeate;
c. hydrolyzing the product obtained in step (b) with an enzyme maintaining pH in the range of 7 to 10 and at temperature in the range of 25 to 75°C for period in the range of 3.5 to 4 hr to obtain hydrolysed product;
d. filtering the hydrolyzed product of step (c) through ultrafiltration membrane at temperature in the range of 15 to 55°C and concentrating the permeate at temperature in the range of 50 to 100°C under vacuum followed by drying by any conventional means known in the art such as spray drying to obtain peptide powder.

In an embodiment of the present invention, said peptide powder exhibit molecular weight less than 2500 Da, with ash content less than 2%.

In an embodiment of the present invention, pH of the solution is adjusted using sodium hydroxide or ammonium hydroxide, preferably ammonium hydroxide.

In another embodiment of the present invention, the peptide solution obtained may be dried by any conventional means known in the art such as spray drying.

In yet another embodiment of the present invention, enzyme used is produced from a non-genetically modified microorganism, preferably a bacterial protease enzyme.

In yet another embodiment of the present invention, bacterial protease enzyme is preferably from *Bacillus* sp., particularly *B.licheniformis.*

In yet another embodiment of the present invention, the ultrafiltration membrane used in steps (b) and (d) is same or different selected from either a polymeric membrane or a ceramic membrane of a known molecular weight cut off, preferably a membrane with 10 kDa or 15 kDa nominal molecular weight cut-off (NMWC) membrane having operational temperature in the range of 1 to 55°C.

In yet another embodiment of the present invention, complete hydrolysis of gelatine to corresponding peptides with retention time at 16.619 min and molecular weight less than 2500 Daltons has been confirmed by size-exclusion chromatography (SEC).

In yet another embodiment of the present invention, yield of the peptide is at least 85%.

In yet another embodiment, present invention provides a composition comprising peptides prepared by the process as claimed in claim 1 with a molecular weight of less than 2500 Da, with ash content less than 2% optionally along with acceptable additives.

In yet another embodiment of the present invention, additives are selected from but not limited to colours, flavours, sweeteners, milk products, flow enhancers and such like.

In yet another embodiment of the present invention, said composition is formulated as powders, flavoured powders, granules, pouches and such like.

In yet another embodiment of the present invention, said composition is useful as peptide supplements for subjects in need of them.

In yet another embodiment of the present invention, said composition inhibits Angiotensin-1 converting enzyme.

### BRIEF DESCRIPTION OF DRAWINGS

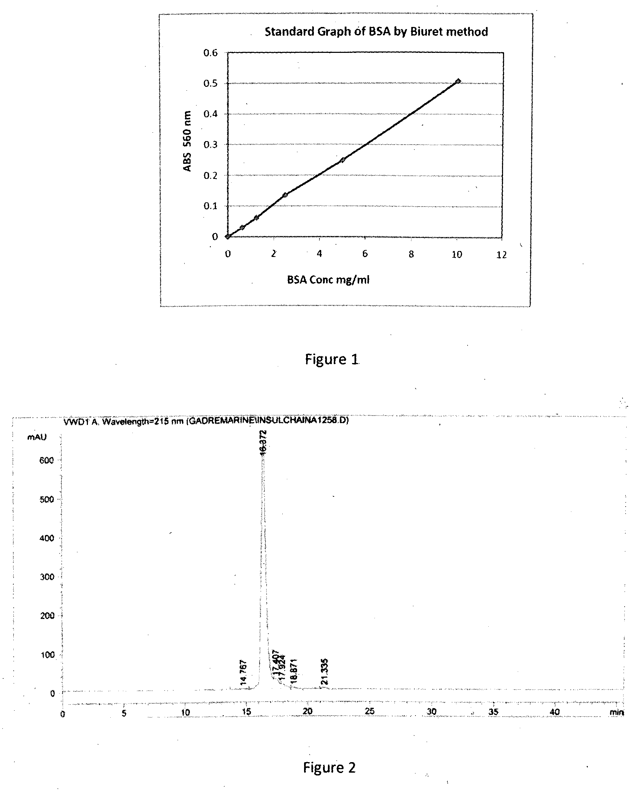

Figure 1 represents standard graph of estimation of bovine serum albumin (BSA) by Biuret method.
Figure 2 represents molecular weight determination by Size exclusion chromatography of insulin. Column: Agilent BioSec, 5 micron, 30°C, Mobile phase: 150 mM Phosphate buffer, pH 7.0, 0.5 ml per min, Detector: UV 215 nm. The chromatogram shows results for standard Insulin Chain A. Molecular weight 2531 Daltons, shows peak at 16.372 min.
Figure 3 represents molecular weight determination by Size exclusion chromatography of raw gelatine under conditions same as for insulin chain A. Figure 3 for raw gelatine from fish collagen- Shows one single peak at 11.016 minutes.
Figure 4 represents size-exclusion chromatography (SEC) for hydrolyzed gelatine with enzyme at 50°C for 15 min and shows major polypeptides produced by enzyme action with retention time (RT) 13.317, 14.116, 14.902, 15.827, 16.424 min within initial 15 min of hydrolysis.
Figure 5 represents SEC for peptides ultra filtered through 10 kDa NMWC membrane and spray dried. All the peaks are later than 16.619 min.

### DETAILED DESCRIPTION OF THE INVENTION

Present inventors provide a process for complete conversion of gelatine to peptides with molecular weights less than 2500 using an enzyme digestion coupled with ultra filtration, concentration and spray drying to obtain the low molecular weight peptide molecules as dry, free flowing peptide powder with high peptide content more than 98% (w/w) and low (less than 2% w/w) salt content.

The process according to the present invention eliminates need for Nano filtration, ion exchange resins or chromatographic processes for peptide purification.

Accordingly, the invention discloses an enzymatic process for the preparation of peptides of molecular weight less than 2500 Daltons from gelatine, wherein nearly half of the peptides in the mixture are of molecular weight less than 1000 Daltons and also have an ash content of less than 2%.

In the present invention, hydrolysis of gelatine is carried out by using an enzyme to obtain low molecular weight peptides of less than 2500 Daltons. The hydrolysis of gelatine is carried out using the enzymes selected from bacterial protease, preferably, using commercially available *Bacillus* protease. The process employs membrane assisted 'gelatine desalting' to removes salts, acids, pigments, fishy odour and low molecular weight compounds from gelatine to begin with.

In accordance with the invention, the process of preparing low molecular weight peptides from gelatine comprises:
a. clarifying the gelatine solution by adjusting pH upto 7 and centrifuging the solution followed by membrane filtration to make the solution particle-free by discarding the solid particles;
b. desalting the solution by filltering through ultra filtration membrane at 20-55°C in a diafiltration mode till the salt content decreases below 0.05% (w/v) followed by discarding the permeate;
c. hydrolyzing the product obtained in step (b) with an enzyme at pH 7-10 and at 25-75°C; and
d. filtering the hydrolyzed product of step (c) through ultrafiltration membrane at 15-55°C and concentrating the permeate at 50-100°C under vacuum to obtain the peptide solution of the invention.

The pH is adjusted using an alkali hydroxide, most preferably ammonium hydroxide.

The concentration of gelatine solution in water may be in the range of 0.5 % w/v to 6.5% w/v.

The peptide solution obtained may be dried by any conventional means known in the art such as spray drying.

The gelatine used for preparing peptides is a fish gelatine solution, which is obtained from Gadre Marine Export Pvt. Ltd. Ratnagiri, Maharashtra, India which is a commercially available source.

The enzyme used in step (c) of the process of the invention is produced from a non-genetically modified microorganism, preferably a bacterial protease enzyme. The most preferred bacterial protease is from *Bacillus* sp., particularly *B.licheniformis.*

The ultrafiltration membrane used in steps (b) and (d) of the process of the invention is same or different and is selected from either a polymeric membrane or a ceramic membrane of a known molecular weight cut off, preferably a membrane with 10 kDa or 15 kDa nominal molecular weight cut-off (NMWC) membrane. The membrane chosen has an operational temperature of 45-55°C. Further, the membrane module may be of a flat sheet, hollow fibre, spiral wound or any suitable configuration.

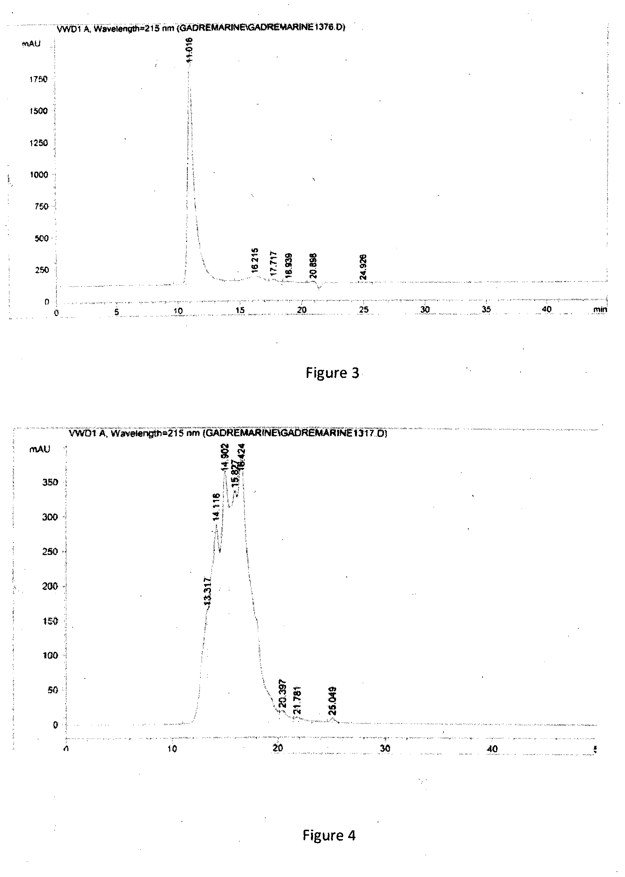

The molecular weight range of the digest mixture was determined using the Agilent 1200 system (Agilent Technologies, Palo Alto, CA, USA). During the digestion process, the retention time of the peptides in the digest mixture increased rapidly in the chromatogram, indicating that larger peptides in the gelatine were rapidly degraded into peptides with lower molecular weight (Figs.3 to 5). The peptides obtained by the process of the invention are of molecular weight less than 2500, with ash content less than 2%. The ash content of the samples was estimated by incineration at 550 °C in a muffle furnace. The molecular weight determination of the peptide is obtained by size exclusion chromatography as exemplified herein by comparing with the retention times of standards such as insulin chain A and vitamin B12.

The comparison of the results obtained are tabulated herein

| **Figure No.** | **Sample** | **Retention time (min)** | **Molecular weight (Dalton)** |
|---|---|---|---|
| 2 | Human Insulin Chain A | 16.372 | 2531 |
| 3 | Fish Gelatine | 11.016 | <100,000 |
| 4 | Gelatine Hydrolysate after 15 Min reaction | 13.317, | 14000, |
| | | 14.116, | 6000, |
| | | 14.902, | 4500, |
| | | 15.827, | 3000, |
| | | 16.424 | 2200 |
| 5 | Peptide product, spray dried | 16.619 and later | Less than 2500 |

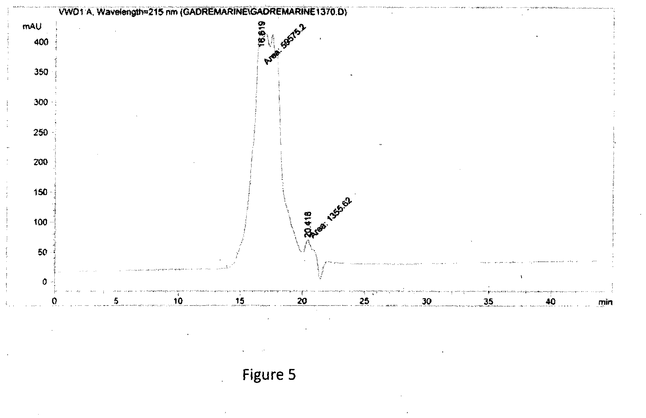

The yield of the peptides according to the process of the invention is at least 85 %. With reference to figure 3, it was observed that the peak for fish gelatine at 11.016 minutes, while in figure 5, there is no peak at 11.016, indicating complete hydrolysis of gelatine to corresponding peptides with retention time at 16.619 and molecular weight less than 2500 Daltons.

In an aspect of the invention, compositions comprising peptides obtained by the process of the invention are disclosed. The composition is formulated as powders, flavoured powders, granules, pouches and such like. Compositions comprising the peptides are useful as peptide supplements for subjects in need of them.

Compositions of the peptides obtained by the process of the invention comprise atleast 70% peptide mixture along with acceptable additives. The additives are selected from, but not limited to colours, flavours, sweeteners, milk products, flow enhancers and such like.

In another aspect of the invention, the composition with atleast 70% w/w peptides obtained by the process of the invention inhibits angiotensin converting enzyme. The peptide inhibits the enzyme when used in the concentration range of 0.125-1 mg/ml.

### EXAMPLES

Following examples are given by way of illustration.

### Example 1

Five litre of fish gelatine solution (obtained from Gad re Marine Export Pvt. Ltd. Ratnagiri, Maharashtra, India) containing 295 g of gelatine was centrifuged at 15000 rpm in a continuous centrifuge to remove insoluble particulate matter and heated to 50 °C and subjected to a desalting step using a polymeric membrane unit of 10 kDa (Nominal molecular weight cut-off) NMWC, at 1 kg/cm² pressure. The high molecular weight of gelatine allows removal of low molecular weight impurities and salts using a ultra filtration process. The volume in retentate tank was maintained by addition of demineralised water continuously. Four volumes of the permeate was collected and analyzed for protein content by Biuret method to ensure no loss of gelatine. The permeate was discarded. The retentate (In an ultrafiltration process, what goes through the membrane (filtrate) is called permeate while the stream containing compounds that do not go through the membrane is called retentate) which contained the desalted gelatine was heated to 60 °C and pH was adjusted to 7.0 with Ammonium Hydroxide and 800 microliters of the enzyme were added to the gelatine solution. The pH of the solution was maintained with an ammonium hydroxide solution at 7.0 till the hydrolysis was completed. Samples were drawn during the reaction at regular interval and analyzed by size exclusion chromatography using a HPLC. When the reaction was completed, the temperature of the solution was decreased to 50 °C and the peptides were recovered by ultra filtration using a 10 kDa NMWC membrane unit. The volume of the retentate was decreased to 500 ml while 4500 ml permeate, the aqueous filtrate, that contained the peptides of low molecular weight was collected. Five hundred ml demineralised water was added twice to the retentate and ultra filtration continued till 5.5 L permeate was collected to ensure recovery of nearly all low molecular weight peptides in to the permeate. The permeate was concentrated under vacuum at 75 °C and then concentrate was spray dried to obtain dry, free flowing, white fish collagen peptide powder with high peptide content and low salt content. The molecular weight of the peptide obtained was less than 2500 Dalton and the ash content was less than 1.5% analysed by incineration of a weighed sample at 550 °C in a muffle furnace. The amino acid profile of the spray dried collagen peptide showed that it is rich in glycine, alanine, proline, lysine, hydroxyproline, and hydroxylysine, characteristic of collagen peptides as detailed in Table below.

Relative amino acid composition of the spray dried fish collagen peptide mixture

| **Amino acid** | **Concentration %** |
|---|---|
| Aspartic acid | 1.59 |
| Glutamic acid | 6.18 |
| Hydroxyproline | 10.03 |
| Serine | 3.10 |
| Glycine | 27.01 |
| Histidine | 0.89 |
| Arginine | 8.21 |
| Threonine | 2.26 |
| Alanine | 13.33 |
| Proline | 11.49 |
| Tyrosine | 0.44 |
| Valine | 2.38 |
| Methionine | 2.34 |
| Isoleucine | 1.06 |
| Leucine | 2.89 |
| Hydroxylysine | 0.43 |
| Phenylalanine | 2.64 |
| Lysine | 3.71 |

### Example 2

### Hydrolysis of gelatine at different pH, under identical conditions

The enzyme chosen has a broad pH range with an optimal at 9. However, on considering the quantity of NaOH required that would 1) remain in the product as a salt and also 2) that the pH of the finished product when dissolved in water would be alkaline the hydrolysis was done at three different pH values. Hydrolysis of gelatine solution (TS 5.92 g/100 ml) at three different pH but otherwise identical conditions was performed. The gelatine solution was placed in a hydrolyzer. The temperature was controlled at 60 °C. pH was adjusted at 7 and 9 in two subsequent experiments. The reaction was continued for 4 hours.

The enzyme was inactivated by increasing temperature to 90°C for 5 min and then cooled to 50 °C. This was done only to correctly measure degree of hydrolysis in 4 h although it is not necessary for process. The hydrolysate was subjected to UF using 3 kDa (Omega Pall Life Science) membranes. The total solids in the permeate and retentate were estimated at 103 °C to constant weight. SEC-HPLC of the peptides produced at different pH values was performed to determine molecular weight of the peptides. It was observed that the amount of alkali required for maintaining pH at 7 was much less as compared to that at 9. pH. The extent of hydrolysis at both pH values was similar and above 86% as shown below. The SEC-HPLC performed showed that there was no difference in the retention times of the peaks of the peptides and therefore had similar molecular weights.

| **pH for Hydrolysis** | **7** | **9** |
|---|---|---|
| Time of Hydrolysis | 4 h | 4 h |
| NaOH required to complete reaction (ml/ 100 ml gelatine | 10.5 | 16.9 |
| Ultra filtration membrane | Omega 3 kDa | Omega 3 kDa |
| Dry Solids in 50 ml initial Gelatine Solution (g/100 ml) | 5.92 | 5.92 |
| Solids in Total Permeate (g/100 ml) | 5.10 | 5.14 |
| Degree of Hydrolysis % | 86.1 | 86.8 |

### Example 3

### Composition:

| | |
|---|---|
| Collagen Peptide powder | 100% |

**Procedure:** The collagen peptide powder is packed in pouches/ bottles/ bags without any additive.

**Mode of administration:** Dissolve 5 gram collagen peptide powder in 25 ml water/ milk/ butter milk/ fruit juice/ soft drink and dissolve by mixing. Collagen peptide powder forms a clear non-bitter solution at this and higher concentrations.

### Example 4

### Composition

| | |
|---|---|
| Collagen Peptide powder | 90.0% (w/w) |
| Colour Erythrosine E 127 | 0.3% (w/w) |
| Strawberry Flavour | 0.7% (w/w) |
| Magnesium stearate | 2.0% (w/w) |
| Mannitol | q.s. (*quantity sufficient*) to 100.0% (w/w) |

Procedure: Dissolve mannitol in water, add colour and flavour to it.

Evaporate water to adsorb colour and flavour on mannitol.

Mix active ingredient and Magnesium stearate and fill in pouch/bottle.

### Mode of administration

Dissolve/ disperse the powder in water/juice. Collagen peptide powder is completely soluble in water.

### Example 5

### Composition

| | |
|---|---|
| Collagen Peptide Powder | 90% (w/w) |
| Instant Coffee Powder | 1% (w/w) |
| Sucrose Powder | 7% (w/w) |
| Magnesium stearate | 2% (w/w) |

**Procedure:** Mix peptide powder, instant coffee powder, sucrose powder and magnesium stearate and fill in pouch/ bottle.

### Mode of administration

Dissolve/ disperse the powder in hot water. Collagen peptide powder is completely soluble in water.

### Example 6

| | |
|---|---|
| Collagen Peptide powder | 70% (w/w) |
| Fresh cream | 15% (w/w) |
| Refined sugar | 12% (w/w) |
| Cocoa powder | 3% (w/w) |

Procedure: Mix collagen peptide powder, fresh cream, refined sugar and cocoa powder and fill in pouch/ boxes/ bottles.

Mode of administration: Mix and use as a topping on Bread/ Cake/ icecream/ milk shakes/ fruit pulps.

### Example 7

Angiotensin-converting enzyme (ACE) Inhibition Screening Kit KT-534 from Kamiya Biomedical Company, 12779 Gateway Drive, Seattle, WA 98168, USA, was used for the analysis of ACE inhibition activity. 20 µl of fish collagen peptide solutions at 1, 0.5, 0.25, 0.125 and 0.0625 mg/ml concentration were mixed with 20 µl substrate buffer and 20 µl enzyme reagent in micro wells of 96 micro well plates with flat bottom. All reactions were performed in duplicate. The micro well plate was incubated at 37 °C for 60 min. At the end of incubation, to each well, 200 µl of the indicator solution was added and mixed well. The plate was incubated further for 10 min at room temperature and the absorbance was measured at 450 nm using a 96 well plate reader.

### Angiotensin-I converting enzyme (ACE) inhibition activity of Fish collagen peptide at different concentrations

| Sr. No. | Concentration mg/ml | Inhibition of ACE % |
|---|---|---|
| 1 | 1.0 | 78.8 |
| 2 | 0.5 | 66.0 |
| 3 | 0.25 | 47.5 |
| 4 | 0.125 | 18.0 |

The results show that the fish collagen peptide caused inhibition of the ACE activity and that the inhibition was proportional to the concentration of the peptide between 0.125 mg/ml and 1 mg/ml.

The fish collagen peptide described in the present document thus shows an additional beneficial activity of Angiotensin 1 converting enzyme inhibition.

### INDUSTRIAL ADVANTAGES OF THE INVENTION

1. The process according to the present invention employs membrane assisted gelatine desalting which removes salts, acids, pigments, fishy odour and low molecular weight compounds from gelatine to begin with.
2. The process of the invention uses a Non-GMO, GRAS protease of bacterial origin from a commercial source that effectively causes hydrolysis of the gelatine by 85-100%.
3. The process utilizes only one enzyme and at a specific pH to make the product that is not acidic or alkaline.
4. The process eliminates need for Nano filtration, or ion exchange resins or chromatographic processes for peptide purification.
5. Use of higher temperature (50°C and above) during desalting, hydrolysis and ultrafiltration for peptide production minimizes problem of microbial contamination and also increases the filtration rate during membrane filtration processes.
6. The process of hydrolysis is accomplished in 4 hours as against many other processes that need long hydrolysis time.
7. The process does not use viable bacteria unlike many other processes.

## Claims

1. A process for preparing low molecular weight peptide comprising the steps of:
a. clarifying 0.5 to 6.5% w/v fish gelatine solution by adjusting pH upto 7 followed by centrifuging the solution followed by membrane filtration to make the solution particle-free by discarding the solid particles;
b. desalting the solution as clarified in step (a) through ultrafiltration membrane at temperature in the range of 20 to 55°C in a diafiltration mode till the salt content decreases below 0.05% (w/v) followed by discarding the permeate;
c. hydrolyzing the product obtained in step (b) with an enzyme maintaining pH in the range of 7 to 10 and at temperature in the range of 25 to 75°C for period in the range of 3.5 to 4 hr to obtain hydrolysed product;
d. filtering the hydrolyzed product of step (c) through ultrafiltration membrane at temperature in the range of 15 to 55°C and concentrating the permeate at temperature in the range of 50 to 100°C under vacuum followed by drying by any conventional means known in the art such as spray drying to obtain peptide powder.

2. The process as claimed in claim 1, wherein said peptide powder exhibit molecular weight less than 2500 Da, with ash content less than 2%.

3. The process according to claim 1, wherein the pH of the solution is adjusted by using sodium hydroxide or ammonium hydroxide, preferably ammonium hydroxide.

4. The process as claimed in step (c) of claim 1, wherein the enzyme used is produced from a non-genetically modified microorganism, preferably a bacterial protease enzyme from *Bacillus* sp., particularly *B.licheniformis.*

5. The process as claimed in claim 1, wherein the ultrafiltration membrane used in steps (b) and (d) is same or different selected from either a polymeric membrane or a ceramic membrane of a known molecular weight cut off, preferably a membrane with 10 kDa or 15 kDa nominal molecular weight cut-off (NMWC) membrane having operational temperature in the range of 1 to 55°C.

6. The process as claimed in claim 1, wherein yield of the peptide is at least 85%.

7. A composition comprising peptides prepared by the process as claimed in claim 1 with a molecular weight of less than 2500 Da, with ash content less than 2% optionally along with acceptable additives selected from colours, flavours, sweeteners, milk products, flow enhancers.

8. The composition as claimed in claim 7, wherein said composition is formulated as powders, flavoured powders, granules; pouches and such like.

9. The composition as claimed in claim 7, wherein said composition is useful as peptide supplements for subjects in need of them.

10. The process as claimed in claim 1, wherein said low molecular weight peptides inhibit Angiotensin-I converting enzyme.

## Patentansprüche

1. Verfahren zur Herstellung von Peptid mit niedrigem molekularen Gewicht, umfassend die Schritte:
a. Klären von 0,5 bis 6,5% w/v Fischgelatinelösung durch Einstellen des pH-Wertes auf bis zu 7, gefolgt von Zentrifugieren der Lösung, gefolgt von Membranfiltration, um die Lösung durch Aussondern der Feststoffpartikel partikelfrei zu machen;
b. Entsalzen der Lösung, wie in Schritt (a) geklärt, durch Ultrafiltrationsmembran bei einer Temperatur im Bereich von 20 bis 55°C in einem Diafiltrationsmodus, bis der Salzgehalt unter 0,05% (w/v) abnimmt, gefolgt von Aussondern des Permeats;
c. Hydrolysieren des in Schritt (b) erhaltenen Produkts mit einem Enzym, unter Beibehalten eines pH-Wertes im Bereich von 7 bis 10 und bei einer Temperatur im Bereich von 25 bis 75°C für einen Zeitraum im Bereich von 3,5 bis 4 Stunden, um hydrolysiertes Produkt zu erhalten;
d. Filtern des hydrolysierten Produkts von Schritt (c) durch Ultrafiltrationsmembran bei einer Temperatur im Bereich von 15 bis 55°C und Konzentrieren des Permeats bei einer Temperatur im Bereich von 50 bis 100°C unter Vakuum, gefolgt von Trocknen mit beliebigen konventionellen Mitteln, die im Stand der Technik bekannt sind, wie beispielsweise Sprühtrocknen, um Peptidpulver zu erhalten.

2. Verfahren nach Anspruch 1, wobei das Peptidpulver ein Molekulargewicht von weniger als 2500 Da, mit einem Aschegehalt von weniger als 2%, aufweist.

3. Verfahren nach Anspruch 1, wobei der pH-Wert der Lösung unter Verwendung von Natriumhydroxid oder Ammoniumhydroxid, vorzugsweise Ammoniumhydroxid, eingestellt wird.

4. Verfahren nach Schritt (c) von Anspruch 1, wobei das verwendete Enzym aus einem nicht genetisch modifizierten Mikroorganismus, vorzugsweise einem bakteriellen Proteaseenzym aus *Bacillus* sp., insbesondere B. *licheniformis,* hergestellt wird.

5. Verfahren nach Anspruch 1, wobei die Ultrafiltrationsmembran, die in Schritten (b) und (d) verwendet wird, gleich oder verschieden ist, und entweder aus einer Polymermembran oder einer Keramikmembran mit bekannter Molekulargewichtsgrenze, vorzugsweise eine Membran mit 10 kDa oder 15 kDA nomineller Molekulargewichts (NMWC) Membran, die Betriebstemperatur im Bereich von 1 bis 55°C aufweist, ausgewählt ist.

6. Verfahren nach Anspruch 1, wobei die Ausbeute des Peptids wenigstens 85% beträgt.

7. Zusammensetzung, umfassend Peptide, die durch das Verfahren nach Anspruch 1 mit einem Molekulargewicht von weniger als 2500 Da, mit einem Aschegehalt von weniger als 2%, gegebenenfalls zusammen mit akzeptablen Zusatzstoffen, ausgewählt aus Farben, Geschmacksstoffen, Süßstoffen, Milchprodukten, Rieselhilfen, hergestellt wird.

8. Zusammensetzung nach Anspruch 7, wobei die Zusammensetzung als Pulver, aromatisierte Pulver, Granulate, Pouches und dergleichen formuliert ist.

9. Zusammensetzung nach Anspruch 7, wobei die Zusammensetzung nützlich ist als Peptid-Ergänzungen für Subjekte, die sie benötigen.

10. Verfahren nach Anspruch 1, wobei die Peptide mit niedrigem Molekulargewicht das Angiotensin-1 umwandelnde Enzym inhibieren.

## Revendications

1. Procédé de préparation d'un peptide de faible masse moléculaire comprenant les étapes de :
a. clarification de 0,5 à 6,5 % p/v d'une solution de gélatine de poisson par ajustement du pH jusqu'à 7 suivi par une centrifugation de la solution suivie par une filtration sur membrane pour rendre la solution dépourvue de particules par un rejet des particules solides ;
b. dessalage de la solution telle que clarifiée dans l'étape (a) à travers une membrane d'ultrafiltration à une température dans la plage de 20 à 55°C dans un mode de diafiltration jusqu'à ce que la teneur en sel diminue en dessous de 0,05 % (p/v) suivi par un rejet du perméat ;
c. hydrolyse du produit obtenu dans l'étape (b) avec une enzyme maintenant le pH dans la plage de 7 à 10 et à une température dans la plage de 25 à 75 °C pendant une durée dans la plage de 3,5 à 4 h pour obtenir un produit hydrolysé ;
d. filtration du produit hydrolysé de l'étape (c) à travers une membrane d'ultrafiltration à une température dans la plage de 15 à 55 °C et concentration du perméat à une température dans la plage de 50 à 100 °C sous vide suivie par un séchage par tout moyen classique connu dans l'art tel que le séchage par pulvérisation pour obtenir une poudre de peptide.

2. Procédé selon la revendication 1, dans lequel ladite poudre de peptide affiche une masse moléculaire de moins de 2500 Da, avec une teneur en cendre de moins de 2 %.

3. Procédé selon la revendication 1, dans lequel le pH de la solution est ajusté en utilisant de l'hydroxyde de sodium ou de l'hydroxyde d'ammonium, de préférence de l'hydroxyde d'ammonium.

4. Procédé selon l'étape (c) de la revendication 1, dans lequel l'enzyme utilisée est produite à partir d'un microorganisme non génétiquement modifié, de préférence une enzyme protéase bactérienne issue de *Bacillus sp.,* en particulier *B. licheniformis.*

5. Procédé selon la revendication 1, dans lequel les membranes d'ultrafiltration utilisées dans les étapes (b) et (d) sont identiques ou différentes et choisies parmi soit une membrane polymérique soit une membrane céramique d'un seuil de coupure de masse moléculaire connu, de préférence une membrane d'un seuil de coupure de masse moléculaire nominal (NMWC) de 10 kDa ou 15 kDa ayant une température de service dans la plage de 1 à 55 °C.

6. Procédé selon la revendication 1, dans lequel le rendement du peptide est d'au moins 85 %.

7. Composition comprenant des peptides préparés par le procédé tel que revendiqué dans la revendication 1, avec une masse moléculaire moyenne de moins de 2500 Da, avec une teneur en cendre de moins de 2 % facultativement conjointement avec des additifs acceptables choisis parmi des colorants, des arômes, des édulcorants, des produits laitiers, des amplificateurs d'écoulement.

8. Composition selon la revendication 7, dans laquelle ladite composition est formulée sous forme de poudres, de poudres aromatisées, de granules, de sachets et similaires.

9. Composition selon la revendication 7, dans laquelle ladite composition est utile comme compléments peptidiques pour des sujets qui en ont besoin.

10. Procédé selon la revendication 1, dans lequel lesdits peptides de faible masse moléculaire inhibent l'enzyme de conversion de l'Angiotensine-1.
